Europäisches Patentamt

European Patent Office

Office européen des brevets

(19)

(11) Numéro de publication: **0 150 714**
**B1**

(12) **FASCICULE DE BREVET EUROPEEN**

(45) Date de publication du fascicule du brevet: **26.10.88**

(51) Int. Cl.⁴: **A 41 B 13/02**

(21) Numéro de dépôt: **85100079.4**

(22) Date de dépôt: **04.01.85**

(54) Couche-culotte à étanchéité améliorée à la ceinture.

(30) Priorité: **06.01.84 FR 8400180**

(43) Date de publication de la demande:
**07.08.85 Bulletin 85/32**

(45) Mention de la délivrance du brevet:
**26.10.88 Bulletin 88/43**

(84) Etats contractants désignés:
**AT BE CH DE GB IT LI NL**

(56) Documents cités:
**DE-A-2 744 400**
**FR-A-2 271 779**
**GB-A-2 002 237**
**US-A-3 049 228**

(73) Titulaire: **BOUSSAC SAINT FRERES B.S.F.**
**Société anonyme dite:**
**12, rue du Vieux Faubourg**
**F-59800 Lille (FR)**

(72) Inventeur: **Kahle, Didier**
**5, Allee Coqueleux**
**F-59910 Bondues (FR)**

(74) Mandataire: **Casalonga, Axel et al**
**BUREAU D.A. CASALONGA OFFICE JOSSE &**
**PETIT Morassistrasse 8**
**D-8000 München 5 (DE)**

Courier Press, Leamington Spa, England.

## Description

La présente invention se rapporte à une coche-culotte, notamment pour enfants en bas âge, selon le préambule de la revendication unique qui est basic sur le document EP—A—0095034.

Dans les couches-culottes connues de ce type, la feuille imperméable, de même que le cousin absorbant, présentent une forme générale sensiblement rectangulaire, avec ou sans échancrures dans les bords latéraux opposés, à la hateur de l'entre-jambes. La feuille imperméable présente une longueur supérieure à la longueur du coussin absorbant, lequel est fixé en position médiane sur la feuille imperméable de manière que ses deux bords transversaux opposés se trouvent en retrait par rapport aux deux bords transveraux opposés de la feuille imperméable.

Si l'étanchéité de ces couches-culottes connues a déjà été améliorée dans la zone d'entre-jambes, par la prévision de moyens élastiques le long des bords latéraux, (voir p.ex. EP-A-0095034), des problèmes d'étanchéité subsistent aux bords transversaux qui constituent la ceinture de la couche-culotte.

On pourrait concevoir une amélioration de l'étanchéité aux bords transversaux de la couche-culotte par un repliage, sur le coussin absorbant, des deux parties de la feuille imperméable dépassant les bords transversaux du coussin. Toutefois, un tel pliage transversal ne peut pas être envisagé sur la machine de fabrication des couches-culottes puisqu'il impliquerait un arrêt de l'avancement des couches-culottes, alors que toutes les autres opérations de fabrication s'effectuent en continu, c'est-à-dire sans aucun arrêt.

La présente invention a pour objet une couche-culotte du type défini ci-dessus permettant une étanchéité améliorée aux bords transversaux, c'est-à-dire à la ceinture, cela sans réduction de la cadence de fabrication et sans complication de la fabrication des couches-culottes.

La couche-culotte conforme à l'invention telle que définie pour la revendication unique comprend une feuille souple imperméable à l'humidité, de forme générale rectangulaire, ayant perpendiculairement à sa longueur deux bords transversaux opposés sensiblement parallès. Cette couche-culotte comprend, en outre, un coussin absorbant ayant deux bords transversaux opposés sensiblement parallèles, ledit coussin étant fixé sur la face supérieure de ladite feuille de manière que ses bords transversaux se trouvent en retrait pair rapport aux deux bords transversaux correspondants de ladite feuille. La couche-culotte comprend, en outre, un voile perméable à l'humidité qui recouvre la face supérieure de l'ensemble de la feuille et du coussin et qui est fixé à la feuille dans la zone non recouverte par le coussin. La couche-culotte comprend, par ailleurs, des moyens élastiques longitudinaux disposés le long des bords longitudinaux de la feuille, et des moyens d'attache pour fermer la couche-culotte à la hauteur de la taille du porteur. Selon l'invention, la couche-culotte comprend de plus pour fixer, avant utilisation de la couche-culotte, dans une zone en retrait par rapport à au moins l'un des bords transversaux du coussin, la partie de ladite feuille et du voile située entre ledit bord transversal du coussin et le bord transversal correspon-dant de ladite feuille, après repliage de ladite partie de feuille sur le coussin autour d'une ligne coïncidant sensiblement avec ledit bord transversal du coussin, pour améliorer l'étanchéité de la couche-culotte à la ceinture, des applications d'adhésif du type autocollable sous la forme de deux traits en zigzag, continus ou discontinus, de part et d'autre du bord transversal du coussin et inversement symétriques par rapport audit bord.

La réalisation desdits moyens de fixation sur la couche-culotte n'exige pas, comme un pliage transversal, un arrêt du mouvement par ailleurs continu de la couche-culotte à travers la machine de fabrication. La couche-culotte est donc fabriquée en continu, sans que la ou les parties de feuille dépassant le ou les bords transversaux du coussin absorbant soient repliées sur ce dernier, et ce n'est qu'au moment de l'utilisation de la couche-culotte que l'on procède audit repliage et à la fixation du ou des rabats repliés sur le dessus du coussin absorbant.

Les applications d'adhésif peuvent se faire en continu comme cela est bien connu dans la fabrication de couches-culottes.

L'adhésif du type autocollable est adhésif qui ne permet à deux parties d'adhérer l'une à l'autre qu'à condition que les deux parties aient été revêtues du même adhésif.

Un avantage particulier de l'invention réside dans la fait que les deux traits en zigzag assurent une fixation même en cas de pliage imprécis.

It est certes déjà connu par le document US—A—3049228 de prévoir des applications d'adhésif du type auto-collable dans la zone de ceinture d'une cullote du type à fermeture par adhésif destiné à être portée par-dessus une couche également du type à fermeture par adhésif, les applications d'adhésif auto-collable étant prévues de manière à permettre, au moment de l'utilisation de la culotte, la fixation d'un rabat ceinture. Cependant, ce rabat de ceinture est replié indépendamment de la couche proprement dite, c'est-à-dire n'enveloppe pas la bord de la couche dans le but d'améliorer l'étanchéité à la ceinture, mais sert à obtenir une ceinture renforcée, ou de permettre l'adaptation de la culotte à des enfants de tailles différentes. Par ailleurs, sur cette culotte, les applications d'adhésif du type auto-collables se présentent sous la forme de traits rectilignes s'étendant perpendiculairement aux bords transversaux de la culotte, de part et d'autre de la ligne de pliage des rabats.

En se référant aux dessins annexés, on va décrire ci-après plus en détail deux modes de réalisation illustratifs et non limitatifs d'une couche-culotte conforme à l'invention; sur les dessins:

la figure 1 est une vue en plan d'une couche-culotte conforme à l'invention telle qu'elle sort de fabrication;

la figure 2 est une vue en plan de la couche-culotte de la figure 1, après repliage et fixation des rabats améliorant l'étanchéité à la ceinture;

la figure 3 est une vue en plan partielle d'un autre mode de réalisation des moyens de fixation des rabats de ceinture sur une couche-culotte conforme à l'invention.

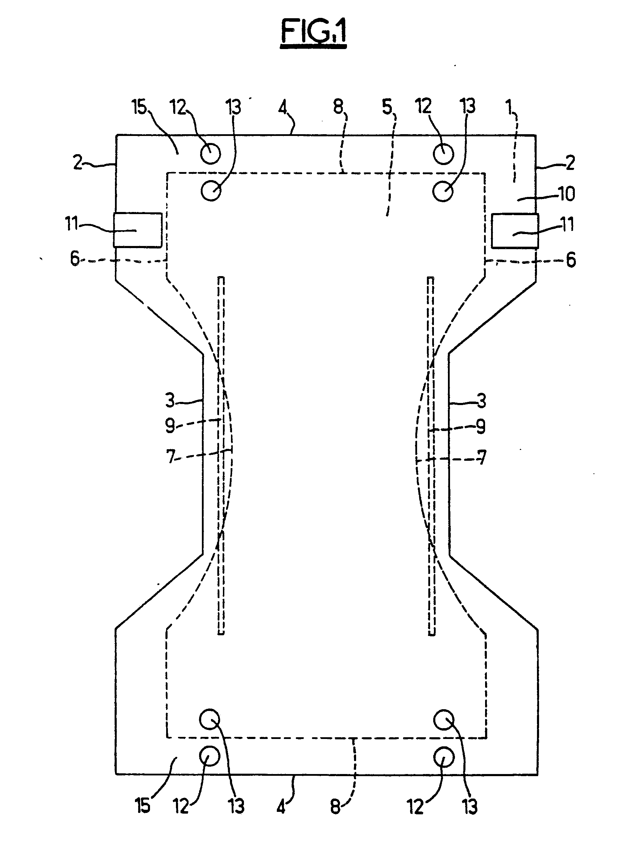

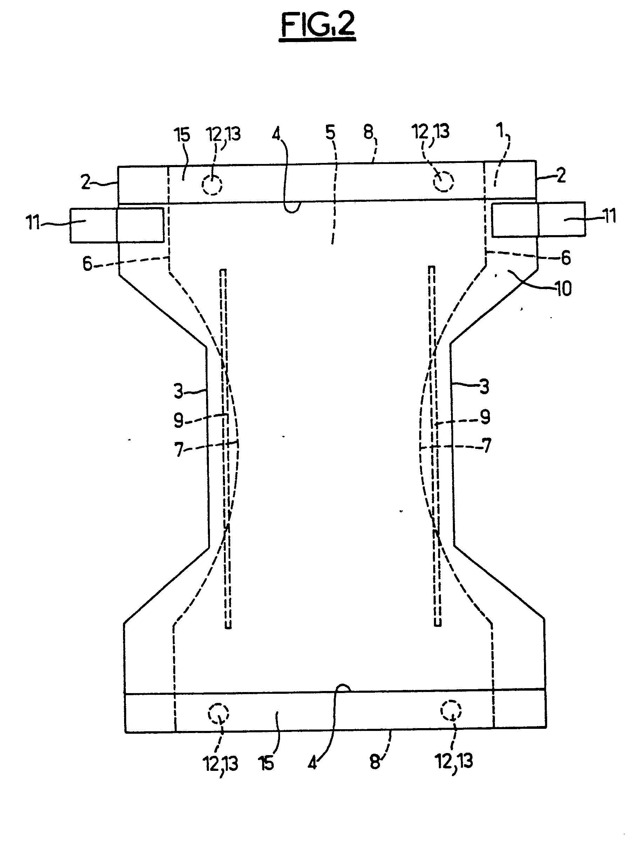

La couche-culotte suivant les figures 1 et 2 est une couche-culotte de structure classique comprenant une feuille 1 souple, imperméable à l'humidité, de forme générale rectangulaire. Les deux côtés latéraux opposés 2 correspondant aux grands côtés du rectangle présentent une échancrure 3 au milieu de leur longueur. Les deux côtés transversaux opposés 4 sont rectilignes.

Sur la face supérieure de la feuille 1 est fixé, par exemple par collage, en position médiane, un coussin absorbant 5 également de forme générale rectangulaire. Le coussin 5 présente une longueur et une largeur inférieures à celles de la feuille 1, de sorte que ses deux bords latéraux opposés 6, munis également chacun d'une échancrure 7 au milieu de leur longueur, et ses deux bords transversaux opposés 8 rectilignes se trouvent en retrait par rapport aux bords correspondants 2 et 4 de la feuille 1.

On reconnaît par ailleurs sur les figures que deux moyens élastiques 9 longitudinaux sont disposés le long des bords latéraux de la feuille 1, dans la zone des échancrures 3. Par ailleurs, l'ensemble de la feuille 1 et du coussin 5 rapporté sur la feuille 1 est recouvert, sur la face supérieure, d'une voile 10 perméable à l'humidité. Enfin, des moyens d'attache 11 par adhésif sont prévus sur les deux bords latéraux 2 de la feuille 1, d'un côté des échancrures 3, en vue de la fermeture de la couche-culotte à hauteur de la taille.

Pour améliorer l'étanchéité de la couche-culotte à la ceinture, c'est-à-dire à la hauteur de la taille, deux paires de points d'adhésifs 12, 13 sont prévues sur la face supérieure de la couche-culotte, sur le voile 10, dans des positions symétriques par rapport à deux lignes qui coïncident pratiquement avec les bords transversaux 8 du coussin 5. Les points 12 de chaque paire de points d'adhésif sont appliqués sur le voile 10 dans les parties 15 comprises entre les bords transversaux 4 de la feuille 1 et les bords transversaux 8 correspondants du coussin 5, et les points 13 sur le voile 10 dans les parties recouvrant le coussin 5, en entrait par rapport aux bords du coussin 5.

Sur la figure 2, les parties 15 de la feuille 1 (et du voile 10) situées entre les bords transversaux 4 de la feuille 1 et les bords transversaux 8 correspondants du coussin 5 ont été repliées sur la face supérieure du coussin 5 autour des bords 8. De ce fait, les points 12 et 13 de chaque paire de points d'adhésif viennent en coïncidence et, sous l'effet d'une pression, adhèrent l'un à l'autre. Les rabats 15 qui améliorent l'étanchéité de la couche-culotte à la hauteur de la ceinture se trouvent ainsi fixés en position repliée.

Ce repliage des rabats 15 n'est effectué qu'au moment de l'utilisation de la couche-culotte. Pour simplifier le repliage correct des rabats 15, il peut être avantageux de rendre nettement visibles les points d'adhésifs 12, 13, par exemple en les collant ou en les entourant d'une trait coloré.

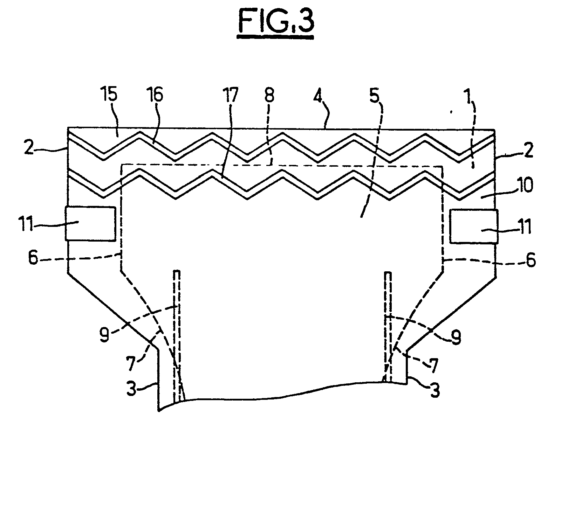

Le mode de réalisation suivant la figure 3 ne diffère du mode de réalisation des figures 1 et 2 que par le remplacement des paires de points d'adhésifs 12, 13 par deux traits d'adhésifs 16, 17 en zigzag disposés de part et d'autre des bords 8. On reconnaît que les deux traits 16 et 17 sont inversement symétriques par rapport aux bords 8.

Ces deux traits d'adhésif 16 et 17 en zigzag permettent de fixer le rabat 15 en position repliée sur le coussin 5 même en cas de repliage imprécis du rabat 15, c'est-à-dire autour d'une ligne autre que le bord 8. Il n'y a donc pas lieu, dans ce mode de réalisation, de prévoir le moindre repère de pliage.

Les deux traits d'adhésif 16 et 17 en zigzag du mode de réalisation de la figure 3, au lieu de s'étendre en continu entre les deux bords latéraux opposés 2 de la feuille 1, pourraient également se présenter sous forme de traits discontinus, par exemple de deux tronçons de trait situés entre chaque bord transversal 2 et le milieu de la largeur de la feuille 1.

**Revendication**

Couche-culotte comprenant une feuille souple (1) imperméable à l'humidité, de forme générale rectangulaire, ayant perpendiculairement à sa longueur deux bords transversaux opposés (4) sensiblement parallèles, un coussin absorbant (5) ayant deux bords transversaux opposés (8) sensiblement parallèles, fixé sur la face supérieure de ladite feuille (1) de manière que ses deux bords transversaux (8) se trouvent en retrait par rapport aux deux bords transversaux (4) de la feuille, un voile (10) perméable à l'humidité, recouvrant la face supérieure de l'ensemble de la feuille (1) et du coussin (5) et fixé à la feuille (1) dans la zone non recouverte par le coussin (5), des moyens élastiques (9) longitudinaux disposés le long des bords longitudinaux de la feuille (1), et des moyens d'attache (11) pour fermer la couche-culotte à hauteur de la taille, caractérisée par le fait qu'elle comprend, en outre, pour fixer avant utilisation, dans une zone en retrait par rapport à au moins l'un des bords transversaux (8) du coussin, la partie (15) de ladite feuille (1) et dudit voile (10) située entre au moins un bord transversal (4) de ladite feuille et le bord transversal correspondant (8) dudit coussin après repliage de ladite partie (15) sur le coussin (5) autour d'une ligne coïncidant sensiblement avec ledit bord transversal (8) du coussin, pour améliorer l'étanchéité de la couche-culotte à la ceinture, des applications d'adhésif du type autocollable, sous la forme de deux traits (16, 17) en zigzag, continus ou discontinus, de part et d'autre du bord transversal (8) du coussin (5) et inversement symétriques par rapport audit bord.

**Patentanspruch**

Windelhöschen, das aus einer weichen, feuchtigkeitsundurchlässigen Folie (1) von im allgemeinen rechteckiger Form mit zweit senkrecht zu ihrer Länge gegenüberliegenden, im wesentlichen parallelen Querrändern (4), einem saugfähigen Kissen (5) mit zwei einander gegenüberliegenden, im wesentlichen parallelen Querrändern (8), das auf der Oberseite der Folie (1) so befestigt ist, daß seine beiden Querränder (8) gegenüber den beiden Querrändern (4) der Folie zurückversetzt sind, einem feuchtigkeitsdurchlässigen Tuch (10), welches die Oberseite der Anordnung aus Folie (1) und Kissen (5) abdeckt und an der Folie (1) in dem vom Kissen (5) nicht bedeckten Bereichbefestigt ist, länglichen elastischen Mitteln (9), die entlang der Längsränder der Folie (1) angeordnet sind, und Verschlüssen (11) zum Schließen des Windelhöschens in der Höhe der Taille besteht, dadurch gekennzeichnet daß es außerdem, um jenen Teil (15) der Folie (1) und des Tuches (10), der zwischen zumindest einem Querrand (4) der Folie und dem entsprechenden Querrand (8) des Kissens liegt, nach Umschlagen des genannten Teils (15) auf das Kissen (5) um eine im wesentlichen mit dem Querrand (8) des Kissens zusammenfallenden Linie, in einem gegenüber zumindest einem der Querränder (8) des Kissens zurückversetzten Bereich vor der Verwendung zu befestigen, zur Verbesserung der Dichtheit des Windelhöschens im Gürtelbereich, selbstklebende Klebstoffaufträge in Form von zwei durchgehenden oder unterbrochenen Zick-Zack-Linien (16, 17) aufweist, die beiderseits des Querrandes (8) des Kissens (5) und zum Rand verkehrt symmetrich verlaufen.

**Claim**

Nappy-pants comprising a flexible sheet (1) impervious to moisture, of generally rectangular shape, having perpendicularly to its length two substantially parallel opposed transverse edges (4), an absorbent pad (5) having two substantially parallel opposed transverse edges (8), which is fixed on the upper face of the said sheet (1) so that its two transverse edges (8) are set back relative to the two transverse edges (4) of the sheet, a moisture-permeable voile (10) covering the upper face of the combination of the sheet (1) and of the pad (5) and fixed to the sheet (1) in the region not covered by the pad (5), lengthwise elastic means (9) arranged along the lengthwise edges of the sheet (1), and fastening means (11) for closing the nappy-pants at waist level, characterized in that they additionally comprise for fixing before use, in a region set back relative to at least one of the transverse edges (8) of the paid, the part (15) of the said sheet (1) and of the said voile (10) situated between at least one transverse edge (4) of the said sheet and the corresponding transverse edge (8) of the said pad, after folding back of the said part (15) on the pad (5) around a line coinciding substantially with the said transverse edge (8) of the pad, to improve the leak-proofing of the nappy-pants at the waist, applications of adhesive of the self-adhesive type, in the form of two continuous or noncontinuous zigzag lines (16, 17) on both sides of the transverse edge (8) of the pad (5) and inversely symmetrical relative to the said edge.

0 150 714

# FIG.1

1

0 150 714

FIG.2

2

# FIG.3